# EUROPEAN PATENT SPECIFICATION

(11) **EP 0 657 466 B1**
(45) Date of publication and mention of the grant of the patent: **15.01.1997**
(21) Application number: 94307839.4
(22) Date of filing: 25.10.1994
(51) Int. Cl.: C07K 1/113, C12N 9/64, B01D 61/14, C07B 63/00

(54) **A process for refolding of (pro-)chymosin, comprising recycling of urea**
Prozess zur Rückfaltung von (pro-)Chymosin, unter Rückführung von Harnstoff
Procédé pour le repliement du (pro-)chymosin, comprenant le recyclage de l'urée

(30) Priority: 10.11.1993 US 150632
(43) Date of publication of application: 14.06.1995
(73) Proprietor: PFIZER INC., New York, N.Y. 10017 (US)
(72) Inventor: Crawford, James G., Terre Haute, Indiana 47802 (US); Stober, Stanley R., Gales Ferry, Connecticut 06335 (US)
(74) Representative: Moore, James William, Dr.

(56) References cited:
- DD-A- 146 751
- HERMANN, R. 'Protein Folding' 1993 , THE EUROPEAN PATENT OFFICE , THE HAGUE, ISBN 90-9006173-8, PAGES 1-6 AND 77-79

## Description

### TECHNICAL FIELD OF THE INVENTION

This invention relates to a process for the recycling of denaturing or solubilizing agents known as chaotropes which are used to solubilize inclusion bodies or insoluble proteins obtained from bacteria using recombinant DNA biotechnology.

### BACKGROUND OF THE INVENTION

Purification schemes for proteins produced by recombinant technology usually involve isolation of insoluble heterologous proteins, also known as inclusion bodies, followed by denaturation and solvation and subsequent renaturation to obtain the biologically active protein. Denaturation is often accomplished by treatment with concentrated solutions of solubilizing agents known as chaotropes; renaturation into the biologically active form of the protein occurs spontaneously when the chaotrope is removed or diluted.

U.S. Patent No. 5,082,775 describes a process for chymosin production which includes the steps of solubilization of inclusion bodies with 8M urea, dilution with an alkaline pH buffer and subsequent neutralization which results in rapid renaturation.

International Patent No. WO 83/04418 describes a process for producing chymosin in which the insoluble form of the chymosin precursor is reversibly denatured with 7M urea or 6M guanidine hydrochloride. The urea or guanidine hydrochloride is then removed by dialysis after which the protein returns to a conformation capable of being converted to active chymosin.

Both of the above procedures when adapted to a commercial processes produce a dilute urea waste solution which results in a difficult and expensive disposal problem.

U.S. Patent No. 5,202,239 discusses solubilization of inclusion bodies containing atrial natriuretic peptide (ANP) with 6M urea. The purified fusion protein was shown to contain proteolytic enzymes from E. coli. These were cleared by contact with immobilized staph V8. ANP was separated from the high molecular weight cleavage products by ultrafiltration over an Amicon YM10 membrane with a 10,000 molecular weight cut off. In addition to ANP, the filtrate stream contained the N-terminal peptide and urea from the cleavage reaction. ANP was separated by cation exchange chromatography or gel filtration chromatography. This reference also describes a number of other peptide substances which are formed as fusion proteins and must undergo a solubilization step during processing. These include urogastrone, proinsulin, and epidermal growth factor.

### SUMMARY OF THE INVENTION

This invention provides an improved process for the preparation of chymosin or prochymosin in which
an insoluble form of said chymosin or prochymosin is produced by E. Coli having a gene coding for said chymosin or prochymosin wherein said insoluble form is solubilized by reversibly denaturing said insoluble form with urea to produce a soluble form of said chymosin or prochymosin, and removing said urea and allowing said solubilized form to renature thereby producing the soluble native form of said chymosin or prochymosin, wherein the improvement comprises:
a) removing at least 50% of said urea from said solubilized denatured form of said chymosin or prochymosin by passing said urea through an ultrafiltration membrane; and
b) adding said urea removed in step a) to supply an effective denaturing amount of urea to an additional quantity of said insoluble form of an additional quantity of said chymosin or prochymosin to produce said soluble form of said chymosin or prochymosin; and periodically cleaning said membrane by:
c) flushing the system at a high flow rate of at least three holdup volumes of pH 10.3 - 10.5 dilution buffer;
d) flushing at a high flow rate with at least 3 holdup volumes of 50°-54°C deionized water;
e) repeating step d) maintaining the pH at 9.5-11.0;
f) adding sodium hypochlorite solution to a free chlorine level of 180-200 ppm;
g) recirculating said hypochlorite solution for 20 to 60 minutes; and
h) flushing with deionized water until said chlorine level is less than 0.15ppm.

The process of this invention may be used in a conventional batch process and is easily adaptable to use in a continuous process such as described in United States Patent No. 4,999,422.

### DETAILED DESCRIPTION OF THE INVENTION

Recombinant DNA procedures have created special problems in the purification of certain proteins from cell extracts and particularly in recovering such proteins in useable forms. These recent developments in recombinant DNA procedures allow the synthesis of foreign proteins in host microorganisms, such as bacterial, yeast and animal cells. This is accomplished by transforming a host cell with a DNA sequence coding for the expression of a foreign protein. When the level of expression of the DNA sequence in a transformed host cell is high, a large amount of the foreign protein is produced within the host cell. Typically the cell sequesters most of these foreign proteins in inclusion bodies within the cytoplasm of the cell. The proteins sequestered in this manner are in the form of insoluble protein aggregates primarily composed of many monomers of the foreign protein bound together, typically through hydrophobic interactions. In this insoluble, aggregate form, the protein is typically not in its preferred biologically active conformation such that the protein is capable of effecting its intended in vivo physiological responses.

Thus, purification of the protein in a biologically active form from the insoluble aggregates requires a means of solubilizing the protein aggregates in such a way to preserve, or to enable ultimate recovery of, the protein in a biologically active conformation.

The initial step in recovering the inclusion body contained proteins from transformed cells generally involves breakage of the cells through a combination of enzyme treatment and mechanical disruption to release the inclusion bodies. Because the protein aggregates contained in the inclusion bodies are insoluble, centrifugation of the resulting cellular material produces a pellet containing a significant amount of the foreign protein, still in the form of insoluble aggregates. The pellet also contains lipids, lipopolysaccharides and traces of nucleic acids.

The next step typically used to recover the protein is to "solubilize" the insoluble protein aggregates. This may be accomplished by treating the membrane pellet with a strong chaotrope, e.g., guanidine hydrochloride, to denature and dissolve the protein. "Solubilization" may also be effected using less stringent chaotropes, e.g., urea that "solubilize" but do not completely denature the desired protein. See e.g., U.S. Pat. No. 4,652,630. The worker of ordinary skill will understand how to select a suitable chaotrope for his particular process.

Solubilizing agents or chaotropes disrupt the protein's conformation factors and "unfold" the protein to a degree that depends on the strength of the solubilizing agent. The greater the extent of the unfolding, the less degree of biological activity the protein likely displays. The resulting solubilized protein solution obtained after one or more of the above-described "solubilizations", thus comprises the foreign protein in some stage of unfolding, depending on the particular solubilizing treatment employed. To obtain a biologically active conformation of the desired protein, it must thus be "refolded". The solubilized protein solution also contains other soluble or solubilized phospholipids, lipopolysaccharides, proteins, and nucleic acids from the inclusion body and insoluble cellular debris. These must, of course, be removed either before or after refolding of the foreign protein.

A typical refolding method used to refold solubilized proteins involves diluting out the solubilizing agent with a large volume of diluent, generally a buffer. When the concentration of solubilizing agent is reduced to a dilution level where the protein's conformation factors begin to reassert themselves, the protein spontaneously refolds into a soluble, biologically active conformation. Depending on the protein, once this 'optimal dilution level" is reached, refolding begins to occur within seconds or may take several minutes or longer.

Typically, the dilution is carried out in one step by mixing the solubilized protein solution with a diluent in a amount necessary to reach the optimal level of dilution. This dilution method is known as a "batch" dilution, drawing its name from the procedure of adding the diluent in one operation to the solubilized protein solution. Utilization of this batch method for refolding a solubilized protein has several disadvantages which are magnified when the refolding is carried out with the large volumes used in commercial scale purification procedures.

Because a solubilized protein solution generally has to be diluted with many times its volume of diluent to achieve at least some degree of refolding, the total volumes being handled at once in commercial protein purification methods can be very large. This process results in large volumes of dilute chaotrope solution which present a serious waste disposal problem.

For example, approximately 5-8M urea is required to solubilize the inclusion bodies (IB) containing the desired protein produced by bacteria. This solution must be diluted by 30-50 fold to allow renaturation to occur. The resulting large volume of dilute urea presents a significant challenge to a waste treatment facility as the volume of the product increases.

There has been a long felt need for a process to recycle a concentrated solution of chaotrope such as urea which is used to solubilize inclusion bodies or insoluble proteins obtained from bacteria or other cells using recombinant DNA technology.

We have found that a concentrated chaotrope solution may be separated from the above-described protein by ultrafiltration and subsequently recycled in a batch or continuous process. For example, urea, with a molecular weight of 60 is very small compared to the desired protein; the ultrafiltration membrane must be chosen with a molecular weight cut off (MWCO) which is larger than urea and smaller than the protein. The membrane must be stable at high pH, about 10-11 for the prochymosin process described herein below. The worker of ordinary skill will be able to select an ultrafiltration membrane which is suited to his particular process.

### SUMMARY OF PROCHYMOSIN PROCESS WITH UREA RECYCLE

Prochymosin inclusion bodies (IB) are dissolved in a 5 to 8M urea solution and then adjusted to an alkaline pH to promote denaturation of the protein. The denatured solution is then concentrated via recirculation of the material to an ultrafiltration (UF) system. The small molecules (urea and water) pass to the permeate. The permeate then is utilized to supply urea to dissolve IB's in a subsequent prochymosin dissolution batch. The UF membranes are cleaned after every batch via a hot water flush, a recirculation hot water alkaline pH wash, a recirculating hot bleach high pH wash, and several more water flushes.

### MEMBRANE SELECTION

In studies with prochymosin, MW about 40,000, the following membranes were investigated for the recycle of the chaotrope urea.

### Koch/Abcor

HFK-131-VSV; 10,000 MWCO, polysulfone
   4 inch spiral
   2 inch spiral
HFK-131-VSV XL-1000, 10,000 MWCO, polysulfone
   4 inch spiral
MSD-328-VS V; 5,000 MWCO, polysulfone
   4 inch spiral
Millipore PTGC; 10,000 MWCO, polysulfone
   2 inch spiral
Millipore PLGC; 10,000 MWCO. celluloric
   2 inch spiral
Millipore PTTK; 30,000 MWCO, polysulfone
   2 inch spiral
IWT Membralox P19-40-200A-Z-E; 20,000 MWCO, ceramic zirconium oxide membrane

The preferred membrane for prochymosin is Koch/Abcor HFK-131-VSV, 10,000 MWCO, polysulfone, 4 inch spiral (Koch Membrane Systems, Inc., 850 Main Street, Wilmington, MA 01887). A worker of ordinary skill enabled by this disclosure would be able to select a membrane for his or her particular system based on considerations such as chaotrope flux, retention of the protein and a repeatable and practical membrane cleaning procedure.

In the prochymosin process, the ultrafiltration (U/F) feed contains about 1% suspended solids. This is a low enough solid content to permit use of spiral U/F systems which offer the advantage of packing the greatest membrane surface area into the least holdup volume. The spiral design and resultant high membrane area to holdup volume ratio facilitates a higher batch concentration factor which is an important design issue.

### MEMBRANE CLEANING

An inclusion body (IB) dissolution stream potentially causes severe and rapid (20-50 minutes) membrane fouling which is cumulative from run to run without proper cleaning. With the prochymosin batch process, the maximum run time was 60 minutes before cleaning was required.

At the end of the batch concentration run, the concentrate is drained and pumped from the U/F system to the renaturation dilution tank. About 10-15 % of the concentrate typically remains behind in the U/F system and recycle tank adhering to the membrane and piping surfaces. To effectively remove this material from the U/F system and not lose the associated product, the system is flushed at high flow rate (350 gpm in a 1900 ft² system) with at least three holdup volumes (a total of about 180 gallons) of pH 10.4 dilution buffer. This flush is directed from the CIP (clean in place) tank through the U/F system to the associated dissolution tank and then to the renaturation tank.

It is important to remove as much of the product at nearly instantaneous dilution rates to achieve the best renaturation yield possible. A slow dilution causes extreme and rapid loss of enzyme activity. Also, adequate flushing is important prior to starting the cleaning cycle since the consumption of chemical cleaning agents is very much a function of how well the system is flushed. The next flush is made with hot 50-54°C deionized water (DIW) to the waste treatment facility. At least three holdup volumes are recommended again at the high flow rate. The conductivity of the effluent from this flush should approach the conductivity of the feed DIW. Next the CIP tank is filled with 50-54°C DIW. Recirculation of this hot DIW is initiated and the pH is adjusted to 10.5 with sodium hydroxide. The system is recirculated (retentate and permeate recycled to the CIP tank) for about 20 minutes while maintaining temperature and pH and then the system is drained to waste treatment.

The system is then refilled with 50-54°C DIW and again the pH is adjusted to 10.5 with sodium hydroxide while the system is recirculating through the U/F. Once the pH and temperature have stabilized, bleach is added until a free chlorine level of 180-200 ppm is reached. Typical bleach consumption is estimated at 20 gallons of 5% bleach for each cleaning of a 1900 Ft² commercial system. Higher bleach concentrations and better flushing will reduce the volume of bleach consumed. The bleach is added in increments and monitored by measuring the free chlorine level. The free chlorine will be consumed as it oxidizes the gel layer on the membrane surface and the free chlorine level will drop. The pH will also drop during the first few bleach additions. When the pH does not drop during the bleach addition, it is a good indicator that the membrane is nearly clean. The liquid in the CIP tank will become clearer (turbidity will decrease) when the membranes are clean. The flux rate on DIW will increase dramatically during the bleach treatment and approach flux rates of 80-100 GFD at TMP of 42.5 psig at 50-55C. The bleach is recirculated for a minimum of 20 minutes and a maximum of 60 minutes. Membrane life is adversely effected by free chlorine levels above 200ppm and pH greater than 11 and temperature greater than 57C. The length of time at any of these conditions should be tightly monitored and controlled. Once the membranes are clean, the bleach solution is drained to waste treatment. The system is then flushed with at least three holdup volumes of 50-54C DIW until a free chlorine level of less than .15 ppm is measured. The CIP tank is then filled with ambient temperature DIW and the system recirculated. This cools the system down prior to filling with the next batch of feed stock and allows for the checking of the DIW flux which is used to benchmark the effectiveness of the cleaning cycle. The system is drained of all DIW just before introducing the next feed stock. It is very important that as much water be drained from the system as possible just prior to filling the system with feed, since the membrane must be kept wet and the water balance on the urea recycle process constrains the amount of permeate that can be recycled.

### EXAMPLE 1

### A. PRODUCTION OF PROCHYMOSIN INCLUSION BODIES

The preparation of prochymosin from genetically modified bacteria is well known in the art. See, for example International Patent WO 83/04418, and United States Patent No. 4,977,248.

Briefly, E. coli is grown in a liquid nutrient medium, then pelleted by centrifugation. The pelleted cells are resuspended in a small volume of buffer and burst open, releasing the prochymosin in the form of densely packed insoluble aggregates (IB's). The aggregates are recovered by a second centrifugation step, while most of the rest of the cellular protein and debris remain in suspension. The prochymosin is thus rapidly purified from the bulk of cellular material. The aggregates may be treated with acid to kill residual cells and to degrade residual DNA. The prochymosin aggregates are then solubilized by denaturation in an alkaline urea solution.

### B. PROCHYMOSIN FEED TO ULTRAFILTRATION UNIT

To 1793 grams of undiluted inclusion body suspension (IB) were added 4440 g of a 10.4M urea solution containing 5.8 g of disodium phosphate. This solution is saturated with respect to urea and has a density of 1.155-1.159 at 23°C. The above suspension was stirred for 90 minutes or until all IB's dissolved. Caustic was then added to increase the pH to 10.3-10.7. This procedure produces a volume of about 5.5 I with a density of 1.129. This liquid is fed to the U/F system and concentrated by a factor of 2.5-3.5.

### C. ULTRAFILTRATION

Flux and yield data were obtained on a Koch/Abcor pilot plant U/F unit that was connected to the discharge line of the IB dissolution tank. The pilot plant consisted of a centrifugal pump capable of delivering a flow of 35 gpm at 64 psig on plant water, a 250 L. recycle tank, and two standard Koch/Abcor four inch diameter spiral housings connected in series. A 200 L permeate surge tank was positioned above the pilot unit to allow for collection of the permeate and easy recombination of the permeate via gravity flow with the concentrate. Retention time did not exceed 75 minutes. Twelve pilot concentration runs and eleven cleaning runs were made to evaluate the flux and retention capabilities of the HFK-131-VSV 10,000 MWCO Koch/Abcor polysulfone membrane. Slip stream runs demonstrated a repeatable membrane cleaning process and that a centrifugal pump can be used in this application with concentration factors as high as 3.3X.

### D. DISSOLUTION RECYCLE

To 3343 gms of recycle permeate that has a density of about 1.116 and a urea concentration of about 7.3M was added 5.8 gms of solid disodium phosphate followed by 1100 gms of solid urea. The mixture was heated at 23°C to dissolve the solid urea in the permeate. This generated a saturated solution of about 10.4 M urea with a density of 1.157-1.159 at 23°C. Running with a saturated liquid urea feed to the 18 dissolution is a good way to control the urea concentration from batch to batch. To 1793 gms of undiluted IB's was added the 10.4 M liquid urea batch prepared above. The mixture was stirred at 20°C for 90 minutes until all IB's were dissolved. Caustic was then added to increase the pH to 10.3-10.7. (A slightly higher pH range 10.3-10.7 is recommended to improve yield.) This procedure generates a dissolution with a volume of about 5.5 L that has a density of 1.129. This material is then fed to the U/F system and concentrated by a factor of 2.5-3.5. The overall average yield of prochymosin for ten iterations was 93.9%.

## Claims

1. An improved process for the preparation of chymosin or prochymosin in which an insoluble form of said chymosin or prochymosin is produced by E. Coli having a gene coding for said chymosin or prochymosin wherein said insoluble form is solubilized by reversibly denaturing said insoluble form with urea to produce a soluble form of said chymosin or prochymosin, and removing said urea and allowing said solubilized form to renature thereby producing the soluble native form of said chymosin or prochymosin, wherein the improvement comprises:
a) removing at least 50% of said urea from said solubilized denatured form of said chymosin or prochymosin by passing said urea through an ultrafiltration membrane; and
b) adding said urea removed in step a) to supply an effective denaturing amount of urea to an additional quantity of said insoluble form of an additional quantity of said chymosin or prochymosin to produce said soluble form of said chymosin or prochymosin; and periodically cleaning said membrane by:
c) flushing the system at a high flow rate of at least three holdup volumes of pH 10.3 - 10.5 dilution buffer;
d) flushing at a high flow rate with at least 3 holdup volumes of 50°-54°C deionized water;
e) repeating step d) maintaining the pH at 9.5-11.0;
f) adding sodium hypochlorite solution to a free chlorine level of 180-200 ppm;
g) recirculating said hypochlorite solution for 20 to 60 minutes; and
h) flushing with deionized water until said chlorine level is less than 0.15ppm.

## Patentansprüche

1. Verbessertes Verfahren zur Herstellung von Chymosin oder Prochymosin, wobei eine unlösliche Form des Chymosins oder Prochymosins durch E. coli, das ein für das Chymosin oder Prochymosin codierendes Gen aufweist, erzeugt wird, die unlösliche Form durch reversible Denaturierung der unlöslichen Form mit Harnstoff zu einer löslichen Form des Chymosins oder Prochymosins solubilisiert wird, und der Harnstoff entfernt und die solubilisierte Form zur Renaturierung belassen wird unter Herstellung der löslichen nativen Form des Chymosins oder Prochymosins, dadurch gekennzeichnet, daß es umfaßt:
a) Entfernen von mindestens 50% des Harnstoffs aus der solubilisierten denaturierten Form des Chymosins oder Prochymosins durch Leiten des Harnstoffs durch eine Ultrafiltrationsmembran und
b) Zugabe des in Schritt a) entfernten Harnstoffs unter Zuführung einer wirksam denaturierenden Harnstoffmenge zu einer weiteren Menge der unlöslichen Form einer zusätzlichen Menge des Chymosins oder Prochymosins zur Herstellung der löslichen Form des Chymosins oder Prochymosins, und periodische Reinigung der Membran durch:
c) Spülen des Systems mit einer hohen Fließgeschwindigkeit von mindestens drei Inhaltsvolumina Verdünnungspuffer von pH 10,3 - 10,5;
d) Spülen bei einer hohen Fließgeschwindigkeit mit mindestens 3 Inhaltsvolumina desionisiertem Wasser von 50°-54°C;
e) Wiederholen von Schritt d) unter Halten des pH-Werts bei 9,5-11,0;
f) Zugabe von Natriumhypochloritlösung mit einem Gehalt an freiem Chlor von 180-200 ppm;
g) Rezirkulation der Hypochloritlösung für 20 bis 60 min und
h) Spülen mit desionisiertem Wasser, bis der Chlorgehalt weniger als 0,15 ppm beträgt.

## Revendications

1. Procédé perfectionné pour la préparation de chymosine ou de prochymosine, dans lequel une forme insoluble de ladite chymosine ou prochymosine est produite par une E. coli ayant un gène codant pour ladite chymosine ou prochymosine, procédé dans lequel ladite forme insoluble est solubilisée en dénaturant de manière réversible ladite forme insoluble avec de l'urée pour produire une forme soluble de ladite chymosine ou prochymosine, en éliminant l'urée et en laissant ladite forme solubilisée subir une renaturation en produisant ainsi la forme naturelle soluble de ladite chymosine ou prochymosine, le perfectionnement consistant :
a) à éliminer au moins 50 % de l'urée de ladite forme dénaturée solubilisée de ladite chymosime ou prochymosine en faisant passer ladite urée à travers une membrane d'ultrafiltration ; et
b) à ajouter ladite urée éliminée dans l'étape a) pour fournir une quantité dénaturante efficace d'urée à une quantité supplémentaire de ladite forme insoluble d'une quantité supplémentaire de ladite chymosine ou prochymosine pour produire ladite forme soluble de ladite chymosine ou prochymosine ; et à nettoyer périodiquement ladite membrane :
c) en balayant le système à un débit élevé d'au moins trois volumes de charge de tampon de dilution à un pH de 10,3 à 10,5 ;
d) en effectuant un balayage à un débit élevé avec au moins trois volumes de charge d'eau désionisée à une température de 50° à 54°C ;
e) en répétant l'étape d) en maintenant le pH dans l'intervalle de 9,5 à 11,0 ;
f) en ajoutant une solution d'hypochlorite de sodium à une teneur en chlore libre de 180 à 200 ppm ;
g) en faisant recirculer ladite solution d'hypochlorite pendant un temps de 20 à 60 minutes ; et
h) en effectuant un balayage avec de l'eau désionisée jusqu'à ce que ladite teneur en chlore soit inférieure à 0,15 ppm.
